# EUROPÄISCHE PATENTSCHRIFT

(11) **EP 1 560 802 B1**
(45) Veröffentlichungstag und Bekanntmachung des Hinweises auf die Patenterteilung: **18.04.2007**
(21) Anmeldenummer: 03810933.6
(22) Anmeldetag: 14.11.2003
(51) Int. Cl.: C07C 45/28, C07C 47/575, B01J 23/70, B01J 23/745

(54) **KATALYTISCHES VERFAHREN ZUR HERSTELLUNG VON CARBONYL-VERBINDUNGEN**
CATALYTIC METHOD FOR THE PRODUCTION OF CARBONYL COMPOUNDS
PROCEDE CATALYTIQUE POUR LA PRODUCTION DE COMPOSES CARBONYLE

(30) Priorität: 14.11.2002 CH 191602; 21.02.2003 CH 273032003
(43) Veröffentlichungstag der Anmeldung: 10.08.2005
(73) Patentinhaber: Clariant Speciality Fine Chemicals (Deutschland) GmbH, 65929 Frankfurt am Main (DE)
(72) Erfinder: ANTOGNOLI, Franco, CH-6005 Luzern (CH); RYS, Paul, CH-8053 Zürich (CH)
(74) Vertreter: Mikulecky, Klaus
(86) Internationale Anmeldenummer: PCT/CH2003/000749
(87) Internationale Veröffentlichungsnummer: WO 2004/043891

(56) Entgegenhaltungen:
- CH-A- 609 318
- K.E. PFITZNER ET.AL.: "SULFOXIDE-CARBODIIMIDE REACTIONS. I. A FACILE OXIDATION OF ALCOHOLS" J. AM. CHEM. SOC., Bd. 87, 1965, Seiten 5661-5670, XP002272909 in der Anmeldung erwähnt

## Beschreibung

Die vorliegende Erfindung betrifft ein katalytisches Verfahren zur Herstellung von aliphatischen und aromatischen Carbonyl-Verbindungen mit mindestens einer Aldehyd- bzw. Ketonfunktion, wobei diese Verbindungen auch gleichzeitig Aldehyd- und Ketonfunktionen aufweisen können.
Die vorstehend genannte Klasse von Verbindungen umfasst nennenswerte Produkte, die im allgemeinen wichtige Zwischen- oder Endprodukte in verschiedenen. Gebieten der sekundären und feinchemischen Industrie darstellen.

Aufgrund der Wichtigkeit der genannten Produkte wurden für deren Herstellung schon viele Verfahren vorgeschlagen. In einigen dieser Verfahren werden als Ausgangsstoffe Alkyl- oder Hydroxyalkyl-Verbindungen verwendet.
In jedem Fall sind jedoch die bisher bekannten Verfahren nicht allgemein verwendbar oder für die industrielle Anwendung zu kompliziert und teuer.
Sie zeigen vor allem dann eine ungenügende Selektivität, wenn es darum geht, Polyaldehyde bzw. Polyketone herzustellen, d.h. Produkte, welche im gleichen Molekül mehrere Aldehyd- bzw. Ketonfunktionen aufweisen.
Verwendet man dafür die bisher bekannten direkten Oxidationsverfahren, wie z.B. beschrieben in den Patenten DE 2605678, CH 609318 oder DE 2944477, dann entstehen gemischt-oxidierte Produkte, bei denen sich am gleichen Molekül kombiniert sowohl Carboxyl-, Hydroxyalkyl- und Formyl- bzw. Ketongruppen befinden.

So beträgt beispielsweise die Selektivität bei der Herstellung von 4,4'-Oxybis(benzaldehyd) aus 4,4'-Oxybis(toluen) nach den bisher bekannten direkten Oxidationsverfahren im allerbesten Fall 30 - 40 mol%.

Ferner ist bekannt, dass Verbindungen mit funktionellen Gruppen der Formel I, 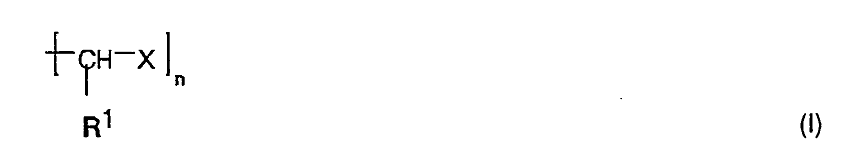 worin R¹ Wasserstoff, Alkyl oder Aryl, X Halogen und n = 1 bedeuten, 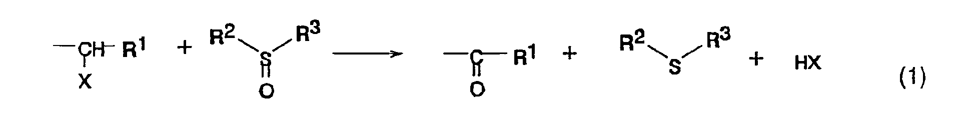 mit Sulfoxiden, worin R² und R³ Alkyl oder Aryl bedeuten, zu Aldehyd- bzw. Ketongruppen und Sulfiden gemäss Reaktion (1) reagieren [Kornblum et al., J. Am. Chem. Soc., 1959, 81, 4113].
In analoger Weise können auch funktionelle Gruppen der Formel I, worin X Hydroxyl bedeutet, mit Sulfoxiden in Aldehyd- bzw. Ketongruppen und Sulfide überführt werden, wenn die Hydroxylgruppe mit einem vorgängig derivatisierten Sulfoxid reagiert, wie z.B. derivatisiert mit Säurechloriden [Omura et al., Tetrahedron 1978, 34, 1651; Mancuso et al., Synthesis, 1981, 165], Dicyclohexylcarbodiimid [Pfitzner et al., J. Am. Chem. Soc., 1965, 87, 5661] oder Säureanhydriden [Albright et al., J. Am. Chem. Soc. 1967, 89, 2416].
Schliesslich ist vor kurzem bekannt geworden, dass funktionelle Gruppen der Formel I, worin R¹ Alkyl oder Aryl und X Wasserstoff bedeuten, mit Sulfoxiden und bei Anwesenheit von Polyoxomolybdaten ebenfalls gemäss Reaktion (1) zu Ketonen und Sulfiden reagieren [Khenkin et al., J. Am. Chem. Soc., 2002, 124, 4198].
In allen diesen Fällen, welche durch Reaktion (1) beschrieben werden, entsteht aus Sulfoxiden die stöchiometrische Menge der entsprechenden Sulfide.

Aufgabe der vorliegenden Erfindung ist es, aliphatische und aromatische Carbonyl-Verbindungen mit hoher Selektivität in einem katalytischen Verfahren herzustellen.

Die Aufgabe wird durch ein katalytisches Verfahren gemäss Patentanspruch 1 gelöst, indem das entstehende Sulfid durch Zugabe eines geeigneten Oxidationsmittels fortlaufend zum entsprechenden Sulfoxid zurückoxidiert wird, so dass das Sulfoxid oder das Sulfid lediglich als Katalysator eingesetzt werden muss.

Das Verfahren wird im Folgenden beschrieben.

Ausgangsstoff dieses katalytischen Verfahrens bildet eine Verbindung, welche mindestens eine (n≥1) funktionelle Gruppe der Formel I, 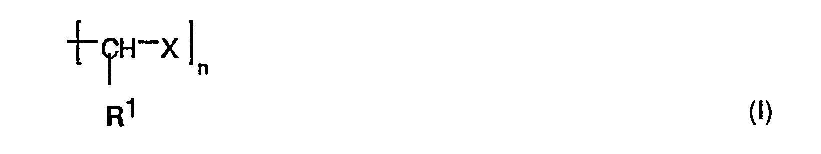 worin R¹ Wasserstoff, Alkyl oder Aryl, X Wasserstoff oder eine während der katalytischen Reaktion gegen die Sulfinylgruppe eines Sulfoxids substituierbare Gruppe und n ganzzahlige Werte zwischen 1 und 8 bedeuten. Diese Verbindung wird in Gegenwart eines Sulfoxids und/oder eines Sulfids bei gleichzeitiger Anwesenheit von Eisensalzen oder Redoxpaaren wie Fe/Cu- oder Ag/Cu-Salzen mittels eines Oxidationsmittels mit einem Redoxpotential von Eₒ > + 2 V vs. NHE (Normal-Wasserstoffelektrode), vorzugsweise mittels eines Persulfatsalzes oxidiert. Durch die Anwesenheit des Sulfoxids und/oder eines Sulfids wird es erst möglich, die gewünschten Carbonylverbindungen mit sehr hoher Selektivität herzustellen, wobei die Bildung von Alkoholen, Carbonsäuren, Dimerisationsprodukten und anderen sekundären Nebenprodukten entscheidend herabgesetzt, bzw. im Wesentlichen verhindert wird.
Diese überraschende Erscheinung des erfindungsgemässen Verfahrens ist der intermediären Ausbildung von funktionellen Gruppen der Formel II zuzuschreiben, 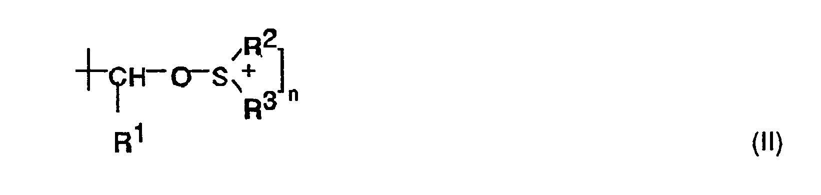 worin R¹ Wasserstoff, Alkyl oder Aryl, R² und R³ Alkyl oder Aryl, n ganzzahlige Werte zwischen 1 und 8 bedeuten. Als Sulfoxide finden Dialkyl-, Alkylaryl- oder Diarylsulfoxide, sowie deren Gemische Anwendung. Als Sulfide finden Dialkyl-, Alkylaryl- oder Diarylsulfide, sowie deren Gemische Anwendung. Die Sulfoxide dienen als Sauerstoff-Transferkatalysatoren, die entweder in der Reaktionslösung löslich oder an einem im Reaktionsgemisch aufgeschlämmten Festkörper immobilisiert sind. Die Sulfide sind entweder in der Reaktionslösung löslich oder an einem im Reaktionsgemisch aufgeschlämmten Festkörper immobilisiert.
Der Stoffmengenanteil des Sulfoxids und/oder des Sulfids beträgt 1 - 90 mol% bezogen auf die gebildete Aldehyd- bzw. Ketonfunktion. Im Weiteren ist auch ein Stoffmengenanteil des Sulfoxids und/oder des Sulfids von 1 - 500 mol% einsetzbar, wenn es z.B. die kinetischen Reaktionsbedingungen erfordern. Auch in diesem Fall kommt der Rückoxidation des intermediär gebildeten Sulfids eine wesentliche Bedeutung zu.
Die Gegenwart eines Sulfoxids und/oder eines Sulfids im Reaktionsgemisch kann in verschiedenster Weise erfolgen.
Das Sulfoxid oder das Sulfid können allein oder in einem Gemisch von Sulfoxiden oder Sulfiden vorliegen. Im Weiteren können das Sulfoxid neben dem Sulfid je einzeln oder je in einem Gemisch vorliegen.
Überraschenderweise führt die Verwendung von mindestens einem Sulfoxid und/oder mindestens einem Sulfid im Reaktionsgemisch zur hohen Selektivität.
Eisensalze allein oder in Kombination mit Silbersalzen dienen primär zur Spaltung des Persulfats zum eigentlichen Oxidationsmittel, dem Sulfat-Radikalanion. Die Verwendung eines Kupfersalzes zusammen mit einem Eisen- bzw. Silbersalz erweist sich als vorteilhaft bei der selektiven Herstellung von Carbonyl-Verbindungen. So wird beispielsweise die Bildung von Dimerisierungsprodukten wirkungsvoll unterdrückt.

Das beschriebene Verfahren kann ausgeführt werden durch graduellen Zusatz des Persulfats in Form von Pulver oder in einer wässrigen Lösung unter heftigem Rühren zum vorgelegten Ausgangsstoff, der in einem inerten organischen Lösungsmittel, in Wasser oder in einem Gemisch davon gelöst ist. Das Sulfoxid und/oder das Sulfid kann in diesem Reaktionsgemisch gelöst oder aufgeschlämmt vorliegen.

Die umfassendsten Bedingungen, welche in Bezug auf den Ausgangsstoff die beste Selektivität ergeben, sind der Zusatz eines Sulfoxids und/oder eines Sulfids und die graduelle Zugabe einer wässrigen Lösung von Persulfat unter heftigem Rühren zu einem Gemisch des Ausgangsstoffes gelöst in Wasser mit einem Anteil an organischen, mit Wasser mischbaren Lösungsmittel. Besonders geeignete Lösungsmittel sind Acetonitril, Methyl- und Ethylalkohol, Aceton, Essigsäure, Dimethylformamid, Acetamid.

Vorzugsweise beträgt der Mengenanteil des organischen Lösungsmittels im Gemisch mit Wasser 25 - 75% bezogen auf das Wasser.

Wirksame Metallsalze sind beispielsweise:
a) Alle wasserlöslichen Eisensalze, vorzugsweise Sulfate, Nitrate und Acetate;
b) alle wasserlöslichen Silbersalze, insbesondere Sulfate, Nitrate und Acetate;
c) alle wasserlöslichen Kupfersalze, vorzugsweise Sulfate, Nitrate und Acetate.

In der Regel beträgt die Konzentration des Eisen- oder Silbersalzes 0.005 -10 mol% bezogen auf den zu oxidierenden Ausgangsstoff. Das Kupfersalz wird in den Redoxpaaren vorzugsweise in einem Molverhältnis von Fe : Cu oder Ag : Cu von 0.1 - 3 eingesetzt.

In der Regel wird die Oxidation bei einer Temperatur von 10 - 100°C ausgeführt.

Erfindungsgemäss kann dem Verfahren auch ein Gemisch von Ausgangsstoffen zu Grunde gelegt werden, wobei eine ähnlich hohe Selektivität an Carbonyl-Verbindungen erzielt wird.

Mit dem erfindungsgemässen katalytischen Oxidationsverfahren kann die Selektivität der hergestellten Carbonyl-Verbindungen unter optimierten Reaktionsbedingungen erheblich, teilweise auf über 90% gesteigert werden.

An Hand der nachstehenden Beispiele wird die vorliegende Erfindung detailliert erläutert, ohne den Anspruch zu erheben, das erfindungsgemässe technische Potential voll beschrieben zu haben, insbesondere sind die Beispiele in keiner Weise optimiert worden.

### Beispiel 1: Herstellung von 4,4'-Oxybis(benzaldehyd)

In einem mit Argon begasten 100 ml Reaktor werden 2 g 4,4'-Oxybis(toluen) (CAS Reg. Nr. 1579-40-4) [10 mmol] in 39.2 ml Acetonitril unter Zusatz von 0.8 ml Dimethylsulfoxid [11.2 mmol] bei 75 °C gelöst. Zur Lösung werden 60 mg Cu(OAc)₂, 30 mg FeSO₄·7 H₂O und 10 ml Wasser beigefügt. 10.8 g Na₂S₂O₈ gelöst in 30 ml Wasser werden anschliessend unter heftigem Rühren zugetropft. Nach 45 Minuten ist die Reaktion beendet. Die organische Phase wird mit Ethylacetat erschöpfend extrahiert. Die gebildeten Produkte werden mit Hilfe der HPLC untersucht.
Die Ausbeute des Dialdehyds 4,4'-Oxybis(benzaldehyd) (CAS Reg. Nr. 2215-76-1) beträgt 87 mol%. Damit liegt diese Ausbeute gegenüber den Ausbeuten mit bisher bekannten Oxidationsverfahren von 30 - 40% wesentlich höher, wodurch sich die Selektivität des erfindungsgemässen Verfahrens auszeichnet.

### Beispiel 2: Herstellung von 4,4'-(1-Methylethan-1,1-diyl)-bis(benzaldehyd)

In einem mit Argon begasten 100 ml Reaktor wurden 2.24 g 1,1'-(1-Methylethan-1,1-diyl)-bis(4-methyl-benzen) (CAS Reg. Nr. 1823-31-0) [10 mmol] in 39.2 ml Acetonitril unter Zusatz von 0.8 ml Dimethylsulfoxid [11.2 mmol] bei 75°C gelöst. Zur Lösung wurden 60 mg Cu(OAc)₂ und 30 mg FeSO₄·7 H₂O und 10 ml Wasser beigefügt. 10.8 g Na₂S₂O₈ gelöst in 30 ml Wasser wurden anschliessend unter heftigem Rühren zugetropft. Nach 100 Minuten ist die Reaktion beendet. Die organische Phase wird mit Ethylacetat erschöpfend extrahiert.
Die Ausbeute des Dialdehyds 4,4'-(1-Methylethan-1,1-diyl)-bis(benzaldehyd) (CAS Reg. Nr. 46948-52-1) beträgt 86 % HPLC.

### Beispiel 3: Herstellung von Biphenyl-4,4'-dicarbaldehyd

In einem mit Argon begasten 100 ml Reaktor wurden 1.82 g 4,4'-Dimethyl-biphenyl (CAS Reg. Nr. 613-33-2) [10 mmol] in 39.2 ml Acetonitril unter Zusatz von 0.8 ml Dimethylsulfoxid [11.2 mmol] bei 70°C gelöst. Zur Lösung wurden 60 mg Cu(OAc)₂ und 50 mg FeSO₄·7 H₂O und 10 ml Wasser beigefügt. 11.8 g Na₂S₂O₈ gelöst in 30 ml Wasser wurden anschliessend unter heftigem Rühren zugetropft. Nach 250 Minuten ist die Reaktion beendet. Die organische Phase wird mit Ethylacetat erschöpfend extrahiert.
Die Ausbeute des Dialdehyds Biphenyl-4,4'-dicarbaldehyd (CAS Reg. Nr. 66-98-8) beträgt 85 mol%.

### Beispiel 4: Herstellung von 4-Methoxy-benzaldehyd

In einem mit Argon begasten 100 ml Reaktor wurden 2.44 g 1-Methoxy-4-methyl-benzen (CAS Reg. Nr. 104-93-8) [20 mmol] in 39 ml Acetonitril unter Zusatz von 1.0 ml Dimethylsulfoxid [14.1 mmol] bei 70°C gelöst. Zur Lösung wurden 65 mg Cu(OAc)₂ und 30 mg FeSO₄·7 H₂O und 10 ml Wasser beigefügt. 11.0 g Na₂S₂O₈ gelöst in 30 ml Wasser wurden anschliessend unter heftigem Rühren zugetropft. Nach 120 Minuten ist die Reaktion beendet.
Die Ausbeute des Monoaldehyds 4-Methoxy-benzaldehyd (CAS Reg. Nr. 123-11-5) beträgt 92 mol%.

### Beispiel 5: Herstellung von 4,4'-Oxybis(benzaldehyd)

In einem mit Argon begasten 100 ml Reaktor werden 2 g 4,4'-Oxybis(toluen) (CAS Reg. Nr. 1579-40-4) [10 mmol] in 39.2 ml Acetonitril unter Zusatz von 0.9 ml Dimethylsulfid [11.0 mmol] bei 75 °C gelöst. Zur Lösung werden 60 mg Cu(OAc)₂, 30 mg FeSO₄·7 H₂O und 10 ml Wasser beigefügt. 16.2 g Na₂S₂O₈ gelöst in 30 ml Wasser werden anschliessend unter heftigem Rühren zugetropft. Nach 65 Minuten ist die Reaktion beendet. Die organische Phase wird mit Ethylacetat erschöpfend extrahiert.
Die Ausbeute des Dialdehyds 4,4'-Oxybis(benzaldehyd) (CAS Reg. Nr. 2215-76-1) beträgt 84 mol%.

### Beispiel 6: Herstellung von 4,4'-Oxybis(benzaldehyd)

In einem mit Argon begasten 100 ml Reaktor werden 2 g 4,4'-Oxybis(toluen) (CAS Reg. Nr. 1579-40-4) [10mmol] in 39.2 ml Acetonitril unter Zusatz von 1.5 ml Methylphenylsulfoxid [11.0 mmol] bei 75 °C gelöst. Zur Lösung werden 60 mg Cu(OAc)₂, 30 mg FeSO₄·7 H₂O und 10 ml Wasser beigefügt. 16.2 g Na₂S₂O₈ gelöst in 30 ml Wasser werden anschliessend unter heftigem Rühren zugetropft. Nach 45 Minuten ist die Reaktion beendet. Die organische Phase wird mit Ethylacetat erschöpfend extrahiert.
Die Ausbeute des Dialdehyds 4,4'-Oxybis(benzaldehyd) (CAS Reg. Nr. 2215-76-1) beträgt 84 mol%.

## Patentansprüche

1. Verfahren zur Herstellung von aliphatischen und aromatischen Carbonyl-Verbindungen mit mindestens einer Aldehyd- bzw. Ketonfunktion, wobei diese Verbindungen auch gleichzeitig mindestens eine Aldehyd- und Ketonfunktion aufweisen können, **dadurch gekennzeichnet, dass** mindestens ein Ausgangsstoff, der mindestens eine aliphatische und / oder aromatisch gebundene funktionelle Gruppe der Formel I 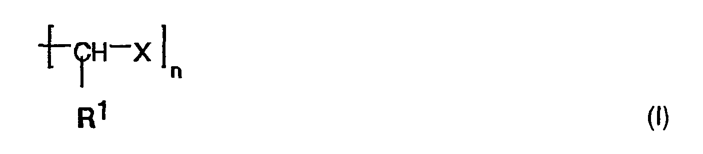 aufweist, worin R¹ Wasserstoff, Alkyl oder Aryl, X Wasserstoff oder eine während der katalytischen Reaktion gegen die Sulfinylgruppe eines Sulfoxids substituierbare Gruppe, n ganzzahlige Werte zwischen 1 bis 8 bedeuten, in Gegenwart mindestens eines Sulfoxids und/oder mindestens eines Sulfids bei gleichzeitiger Anwesenheit von Eisensalzen oder Redoxpaaren von Eisen/Kupfer- oder Silber/Kupfersalzen mittels mindestens eines Oxidationsmittels mit einem Redoxpotential von Eₒ > + 2 V vs. NHE, oxidiert wird, wobei das Sulfoxid und/oder das Sulfid in katalytischer Funktion verwendet wird.

2. Verfahren nach Anspruch 1, **dadurch gekennzeichnet, dass** zur Herstellung von aliphatischen und aromatischen Mono- und Polyaldehyden mindestens ein Ausgangsstoff oxidiert wird, der mindestens eine aliphatisch und / oder aromatisch gebundene funktionelle Gruppe der Formel I aufweist, worin R¹ Wasserstoff bedeutet.

3. Verfahren nach Anspruch 1, **dadurch gekennzeichnet, dass** zur Herstellung von aliphatischen und aromatischen Mono- und Polyketonen mindestens ein Ausgangsstoff oxidiert wird, der mindestens eine aliphatisch und / oder aromatisch gebundene funktionelle Gruppe der Formel I aufweist, worin R¹ Alkyl oder Aryl bedeuten.

4. Verfahren nach Anspruch 1, **dadurch gekennzeichnet, dass** zur Herstellung von Carbonyl-Verbindungen, die sowohl aliphatisch oder aromatisch gebundene Aldehydals auch aliphatisch oder aromatisch gebundene Ketonfunktionen aufweisen, mindestens ein Ausgangsstoff oxidiert wird, der mindestens eine aliphatische und / oder aromatisch gebundene funktionelle Gruppe der Formel I aufweist, worin R¹ bei der Bildung von Aldehydfunktionen Wasserstoff und bei der Bildung von Ketonfunktionen Alkyl oder Aryl bedeutet.

5. Verfahren nach einem der Ansprüche 1 - 4, **dadurch gekennzeichnet, dass** als Sulfoxide Dialkyl-, Diaryl- oder Alkylarylsulfoxide und dass als Sulfide Dialkyl-, Diaryl- oder Alkylarylsulfide verwendet werden.

6. Verfahren nach einem der Ansprüche 1 - 5, **dadurch gekennzeichnet, dass** mindestens ein Sulfoxid und/oder mindestens ein Sulfid im Reaktionsgemisch verwendet wird.

7. Verfahren nach einem der Ansprüche 1 - 6, **dadurch gekennzeichnet, dass** das mindestens eine Sulfoxid und/oder das mindestens eine Sulfid im Reaktionsgemisch gelöst wird.

8. Verfahren nach einem der Ansprüche 1 - 6, **dadurch gekennzeichnet, dass** das mindestens eine Sulfoxid und/oder das mindestens eine Sulfid auf einem Festkörper immobilisiert und dieser im Reaktionsgemisch aufgeschlämmt wird.

9. Verfahren nach einem der Ansprüche 1 - 8, **dadurch gekennzeichnet, dass** das mindestens eine Sulfoxid und/oder das mindestens eine Sulfid mit einem Stoffmengenanteil von 1 - 90 mol% bezogen auf die gebildete Aldehyd- bzw. Ketonfunktion verwendet wird.

10. Verfahren nach einem der Ansprüche 1 - 8, **dadurch gekennzeichnet, dass** das mindestens eine Sulfoxid und/oder das mindestens eine Sulfid mit einem Stoffmengenanteil von 1 - 500 mol% bezogen auf die gebildete Aldehyd- bzw. Ketonfunktion verwendet wird.

11. Verfahren nach einem der Ansprüche 1 -10, **dadurch gekennzeichnet, dass** das Oxidationsmittel in Form von Pulver oder in einer wässrigen Lösung eingesetzt wird.

12. Verfahren nach einem der Ansprüche 1-11, **dadurch gekennzeichnet, dass** als Oxidationsmittel Persulfatsalze oder ein Gemisch davon verwendet wird.

13. Verfahren nach Anspruch 12, **dadurch gekennzeichnet, dass** als Persulfatsalze Alkali- oder Ammoniumpersulfat verwendet werden.

14. Verfahren nach einem der Ansprüche 1-13, **dadurch gekennzeichnet, dass** wasserlösliche Eisensalze, vorzugsweise aus der Gruppe Sulfate, Nitrate und Acetate, allein oder im Gemisch mit wasserlöslichen Kupfersalzen verwendet werden.

15. Verfahren nach einem der Ansprüche 1-13, **dadurch gekennzeichnet, dass** wasserlösliche Silbersalze, vorzugsweise aus der Gruppe Sulfate, Nitrate und Acetate, allein oder im Gemisch mit wasserlöslichen Kupfersalzen verwendet werden.

16. Verfahren nach einem der Ansprüche 1 - 13, **dadurch gekennzeichnet, dass** wasserlösliche Kupfersalze, vorzugsweise aus der Gruppe Sulfate, Nitrate und Acetate, allein oder im Gemisch mit Eisen- oder Silbersalzen verwendet werden.

17. Verfahren nach einem der Ansprüche 1 - 16, **dadurch gekennzeichnet, dass** Eisen- oder Silbersalze in den Redoxpaaren in Konzentrationen von 0.005 - 10 mol% bezogen auf den zu oxidierenden Ausgangsstoff eingesetzt werden und dass das Kupfersalz in einem Molverhältnis von Fe : Cu oder Ag : Cu von 0.1 - 3 eingesetzt wird.

18. Verfahren nach einem der Ansprüche 1 - 17, **dadurch gekennzeichnet, dass** die Oxidation in einem inerten Reaktionsmedium aus der Gruppe Wasser, organisches Lösungsmittel und einem Gemisch davon, ausgeführt wird.

19. Verfahren nach Anspruch 18, **dadurch gekennzeichnet, dass** als organisches Lösungsmittel Acetonitril, Methyl- und Ethylalkohol, Aceton, Essigsäure, Dimethylformamid oder Acetamid verwendet wird.

20. Verfahren nach einem der Ansprüche 1-19, **dadurch gekennzeichnet, dass** die Oxidation bei einer Temperatur von 10 - 100°C ausgeführt wird.

## Claims

1. A process for preparing aliphatic and aromatic carbonyl compounds having at least one aldehyde or ketone function, where these compounds may also simultaneously have at least one aldehyde and ketone function, which comprises oxidizing at least one starting material which has at least one aliphatically and/or aromatically bonded functional group of the formula I 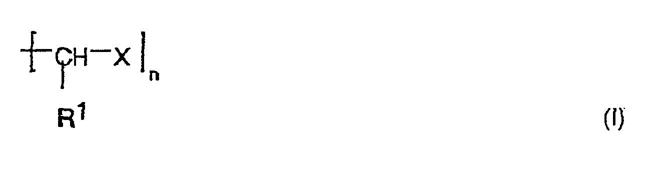 in which R¹ is hydrogen, alkyl or aryl, X is hydrogen or a group which is substitutable for the sulfinyl group of a sulfoxide during the catalytic reaction, n is integer values from 1 to 8, in the presence of at least one sulfoxide and/or of at least one sulfide in the simultaneous presence of iron salts or redox pairs of iron/copper or silver/copper salts by means of at least one oxidizing agent with a redox potential of E₀ of > + 2 V vs. NHE, the sulfoxide and/or the sulfide being used in a catalytic function.

2. The process as claimed in claim 1, wherein aliphatic and aromatic mono- and polyaldehydes are prepared by oxidizing at least one starting material which has at least one aliphatically and/or aromatically bonded functional group of the formula I in which R¹ is hydrogen.

3. The process as claimed in claim 1, wherein aliphatic and aromatic mono- and polyketones are prepared by oxidizing at least one starting material which has at least one aliphatically and/or aromatically bonded functional group of the formula I in which R¹ is alkyl or aryl.

4. The process as claimed in claim 1, wherein carbonyl compounds which have both aliphatically or aromatically bonded aldehydes and aliphatically or aromatically bonded ketone functions are prepared by oxidizing at least one starting material which has at least one aliphatically and/or aromatically bonded functional group of the formula I in which R¹ is hydrogen for the formation of aldehyde functions and is alkyl or aryl for the formation of ketone functions.

5. The process as claimed in one of claims 1 - 4, wherein the sulfoxides used are dialkyl sulfoxides, diaryl sulfoxides or alkyl aryl sulfoxides, and wherein the sulfides used are dialkyl sulfides, diaryl sulfides or alkyl aryl sulfides.

6. The process as claimed in one of claims 1 - 5, wherein at least one sulfoxide and/or at least one sulfide is used in the reaction mixture.

7. The process as claimed in one of claims 1 - 6, wherein the at least one sulfoxide and/or the at least one sulfide is dissolved in the reaction mixture.

8. The process as claimed in one of claims 1 - 6, wherein the at least one sulfoxide and/or the at least one sulfide is immobilized on a solid and this solid is slurred in the reaction mixture.

9. The process as claimed in one of claims 1 - 8, wherein the at least one sulfoxide and/or the at least one sulfide is used in a quantitative proportion of 1 - 90 mol% based on the aldehyde or ketone function formed.

10. The process as claimed in one of claims 1 - 8, wherein the at least one sulfoxide and/or the at least one sulfide is used in a quantitative proportion of 1 - 500 mol% based on the aldehyde or ketone function formed.

11. The process as claimed in one of claims 1 - 10, wherein the oxidizing agent is used in the form of powder or in an aqueous solution.

12. The process as claimed in one of claims 1 - 11, wherein the oxidizing agent used comprises persulfate salts or a mixture thereof.

13. The process as claimed in claim 12, wherein the persulfate salts used are alkali metal persulfate or ammonium persulfate.

14. The process as claimed in one of claims 1 - 13, wherein water-soluble iron salts, preferably from the group of sulfates, nitrates and acetates, are used alone or in a mixture with water-soluble copper salts.

15. The process as claimed in one of claims 1 - 13, wherein water-soluble silver salts, preferably from the group of sulfates, nitrates and acetates, are used alone or in a mixture with water-soluble copper salts.

16. The process as claimed in one of claims 1 - 13, wherein water-soluble copper salts, preferably from the group of sulfates, nitrates and acetates, are used alone or in a mixture with iron or silver salts.

17. The process as claimed in one of claims 1 - 16, wherein iron or silver salts are used in the redox pairs in concentrations of 0.005 - 10 mol% based on the starting material to be oxidized, and the copper salt is used in a molar ratio of Fe : Cu or Ag : Cu of 0.1 - 3.

18. The process as claimed in one of claims 1 - 17, wherein the oxidation is performed in an inert reaction medium from the group of water, organic solvent and a mixture thereof.

19. The process as claimed in claim 18, wherein the organic solvent used is acetonitrile, methyl and ethyl alcohol, acetone, acetic acid, dimethylformamide or acetamide.

20. The process as claimed in one of claims 1 - 19, wherein the oxidation is performed at a temperature of 10 - 100°C.

## Revendications

1. Procédé de préparation de composés carbonylés aliphatiques et aromatiques ayant au moins une fonction aldéhyde ou cétone, ces composés pouvant aussi et simultanément comporter au moins une fonction aldéhyde et cétone, **caractérisé en ce qu'**on oxyde au moins une matière de départ qui comprend au moins un groupe fonctionnel aliphatique et/ou à liaison aromatique, de formule I 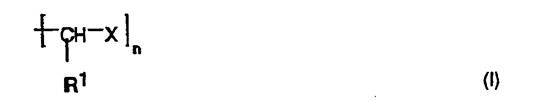 dans laquelle R¹ est un atome d'hydrogène ou un groupe alkyle ou aryle, X est un atome d'hydrogène ou un groupe pouvant, pendant la réaction catalytique, remplacer le groupe sulfinyle d'un sulfoxyde, n est un nombre entier compris entre 1 et 8, en présence d'au moins un sulfoxyde et/ou d'un sulfure et simultanément en présence de sels de fer ou de paires rédox de sels de fer/cuivre ou d'argent/cuivre, à l'aide d'au moins un oxydant ayant un potentiel rédox Eₒ > +2V EHN, le sulfoxyde et/ou le sulfure étant utilisés en fonction catalytique.

2. Procédé selon la revendication 1, **caractérisé en ce que**, pour préparer des mono- et polyaldéhydes aliphatiques et aromatiques, on oxyde au moins une substance de départ qui comprend au moins un groupe fonctionnel à liaison aliphatique et/ou aromatique de formule I dans laquelle R¹ est un atome d'hydrogène.

3. Procédé selon la revendication 1, **caractérisé en ce que**, pour préparer des mono- et des polycétones aliphatiques et aromatiques, on oxyde au moins une substance de départ qui comprend au moins un groupe fonctionnel à liaison aliphatique et/ou aromatique de formule I dans laquelle R¹ est un groupe alkyle ou aryle.

4. Procédé selon la revendication 1, **caractérisé en ce que**, pour préparer des composés carbonylés qui comprennent tant des fonctions aldéhyde à liaison aliphatique ou aromatique que des fonctions cétone à liaison aliphatique ou aromatique, on oxyde au moins une substance de départ qui comprend un groupe fonctionnel aliphatique et/ou à liaison aromatique de formule I dans laquelle R¹, lors de la formation de fonctions aldéhyde, est un atome d'hydrogène et, lors de la formation de fonctions cétone, est un groupe alkyle ou aryle.

5. Procédé selon l'une des revendications 1 à 4, **caractérisé en ce qu'**on utilise en tant que sulfoxydes des dialkyl-, des diaryl-, ou des alkylarylsulfoxydes, et qu'on utilise en tant que sulfures des sulfures de dialkyle, de diaryle ou d'alkylaryle.

6. Procédé selon l'une des revendications 1 à 5, **caractérisé en ce qu'**on utilise au moins un sulfoxyde et/ou au moins un sulfure dans le mélange réactionnel.

7. Procédé selon l'une des revendications 1 à 6, **caractérisé en ce que** le ou les sulfoxydes et/ou le ou les sulfures sont dissous dans le mélange réactionnel.

8. Procédé selon l'une des revendications 1 à 6, **caractérisé en ce que** le ou les sulfoxydes et/ou le ou les sulfures sont immobilisés sur un corps solide, et ce dernier est mis en suspension dans le mélange réactionnel.

9. Procédé selon l'une des revendications 1 à 8, **caractérisé en ce que** le ou les sulfoxydes et/ou le ou les sulfures sont utilisés selon une proportion de 1 à 90 % en moles par rapport à la fonction aldéhyde ou cétone formée.

10. Procédé selon l'une des revendications 1 à 8, **caractérisé en ce que** le ou les sulfoxydes et/ou le ou les sulfures sont utilisés selon une proportion de 1 à 500 % en moles par rapport à la fonction aldéhyde ou cétone formée.

11. Procédé selon l'une des revendications 1 à 10, **caractérisé en ce que** l'oxydant est utilisé sous forme d'une poudre ou en solution aqueuse.

12. Procédé selon l'une des revendications 1 à 11, **caractérisé en ce qu'**on utilise en tant qu'oxydant des sels persulfates ou un mélange de ceux-ci.

13. Procédé selon la revendication 12, **caractérisé en ce qu'**on utilise en tant que sels persulfates un persulfate d'un métal alcalin ou du persulfate d'ammonium.

14. Procédé selon l'une des revendications 1 à 13, **caractérisé en ce qu'**on utilise des sels de fer, de préférence choisis dans l'ensemble comprenant les sulfates, les nitrates et les acétates, à titre individuel ou en mélange avec des sels de cuivre solubles dans l'eau.

15. Procédé selon l'une des revendications 1 à 13, **caractérisé en ce qu'**on utilise des sels d'argent solubles dans l'eau, de préférence choisis dans l'ensemble comprenant les sulfates, les nitrates et les acétates, à titre individuel ou en mélange avec des sels de cuivre solubles dans l'eau.

16. Procédé selon l'une des revendications 1 à 13, **caractérisé en ce qu'**on utilise des sels de cuivre solubles dans l'eau, choisis de préférence dans l'ensemble comprenant les sulfates, les nitrates et les acétates, à titre individuel ou en mélange avec des sels de fer ou d'argent.

17. Procédé selon l'une des revendications 1 à 16, **caractérisé en ce qu'**on utilise des sels de fer ou d'argent dans les paires rédox à des concentrations de 0,005 à 10 % en moles par rapport à la matière de départ devant être oxydée, et qu'on utilise le sel de cuivre selon un rapport en moles Fe:Cu ou Ag:Cu de 0,1 à 3.

18. Procédé selon l'une des revendications 1 à 17, **caractérisé en ce que** l'oxydation est réalisée dans un milieu réactionnel inerte choisi dans l'ensemble comprenant l'eau, un solvant organique et un mélange de ceux-ci.

19. Procédé selon la revendication 18, **caractérisé en ce qu'**on utilise en tant que solvant organique de l'acétonitrile, de l'alcool méthylique et éthylique, de l'acétone, de l'acide acétique, du diméthylformamide ou de l'acétamide.

20. Procédé selon l'une des revendications 1 à 19, **caractérisé en ce que** l'oxydation est mise en oeuvre à une température de 10 à 100°C.
